Europäisches Patentamt

European Patent Office

Office européen des brevets

(11) Publication number: **0 338 815**
**A1**

## (12) EUROPEAN PATENT APPLICATION

(21) Application number: 89303907.3

(22) Date of filing: 19.04.89

(51) Int. Cl.⁴: **A 61 F 13/04**

(30) Priority: 20.04.88 IE 1186/88

(43) Date of publication of application:
25.10.89 Bulletin 89/43

(84) Designated Contracting States:
AT BE CH DE ES FR GB GR IT LI LU NL SE

(71) Applicant: McMahon, Patrick Joseph
7 Brendan House Brendan Road
Donnybrook Dublin 4 (IE)

(72) Inventor: McMahon, Patrick Joseph
7 Brendan House Brendan Road
Donnybrook Dublin 4 (IE)

(74) Representative: Jenkins, Peter David et al
PAGE, WHITE & FARRER 54 Doughty Street
London WC1N 2LS (GB)

(54) Apparatus and process for producing an orthopaedic splint material.

(57) The invention provides a thermoplastic orthopaedic splint material comprising a flexible inert substrate (14) embedded in a thermoplastic material (20). A process for the production of the splint material comprises the steps of coating an inert flexible substrate (14) with a layer of thermoplastic material (20) which is at or above its melting temperature, and cooling the combined substrate and thermoplastic material below the melting temperature of the thermoplastic material. An apparatus for producing the splint material comprises means (15,16) for coating an inert flexible substrate (14) with a layer of thermoplastic material (20) and means for cooling (25) the combined substrate and thermoplastic material below the melting temperature of the thermoplastic material.

FIGURE I

**Description**

**Apparatus and Process for Producing an Orthopaedic Splint Material**

The present invention relates to an apparatus and process for producing a thermoplastic orthopaedic splint material.

Thermoplastic orthopaedic splint materials are known which generally comprise polycaprolactone, a filler and a whitener. The filler usually comprises approximately 20% by weight of the material and the whitener for example titanium dioxide comprises approximately 5% by weight of the material. The known orthopaedic splint materials are also chemically cross-linked to facilitate perforation and also to provide a desired high viscosity of the material to prevent it from "running" when it is softened by heating. The cross-linking is usually achieved by irradiation of the material with gamma rays or an electron beam (2.5 megarad dosage). A chemical catalyst is sometimes also used in conjunction with the irradiation.

One disadvantage of the known type of thermoplastic orthopaedic splint material is that the cross-linking stage of the production process is very expensive requiring the use of irradiation equipment. In addition, the known splint material is produced in sheets which must have a certain large thickness to prevent the material from being too flowable when heated to and above its critical or melting temperature. However, the thickness of the sheet material causes a problem in that the sheets are not as mouldable as many users would desire.

One object of the present invention is to mitigate the above disadvantages of thermoplastic orthopaedic splint materials.

According to the invention there is provided a thermoplastic orthopaedic splint material comprising a flexible inert substrate embedded in a thermoplastic material.

The thermoplastic material is preferably a non-cross-linked polymer. In a preferred form of the invention the thermoplastic material comprises polycaprolactone and the substrate comprises a fabric material. In a particularly preferred form of the invention the thermoplastic material comprises approximately 90% to 99% by weight of polycaprolactone and approximately 1% to 10% by weight of a compatible wax, and more particularly approximately 94% to 97% by weight of polycaprolactone and approximately 3% to 6% by weight of a compatible wax. In a particularly preferred form of the invention the thermoplastic material comprises approximately 95% by weight of polycaprolactone and approximately 5% by weight of a compatible wax.

The invention also provides a process for the production of a thermoplastic orthopaedic splint material comprising, coating an inert flexible substrate with a layer of thermoplastic material which is at or above its melting temperature, and cooling the combined substrate and thermoplastic material below the melting temperature of the thermoplastic material.

The invention further provides an apparatus for the production of a thermoplastic orthopaedic splint material comprising means for coating an inert flexible substrate with a layer of thermoplastic material which is at or above its melting temperature, and means for cooling the combined substrate and thermoplastic material below the melting temperature of the thermoplastic material.

The substrate is preferably a fabric material and the thermoplastic material is preferably polycaprolactone. The thermoplastic material preferably comprises approximately 90% to 99% by weight of polycaprolactone and approximately 1% to 10% by weight of compatible wax. In one preferred form of the process of the invention, the thermoplastic material comprises approximately 94% to 97% by weight of polycaprolactone and approximately 3% to 6% by weight of a compatible wax. In a particularly preferred form of the process of the invention, the thermoplastic material comprises approximately 95% by weight of polycaprolactone and approximately 5% by weight of a compatible wax.

The process is preferably continuous and a head of the thermoplastic material is maintained at a temperature above the melting temperature and is continuously applied to a moving sheet of fabric material. The fabric material is preferably drawn through a pair of draw rollers which are spaced apart to determine the thickness of the eventual sheet of material.

Preferably, a pair of spaced apart guide members are disposed substantially vertically above the draw rollers, the fabric material also being drawn between the guide members, and the thermoplastic material being applied to the fabric material in the region between the guide members.

One of the guide members is preferably heated to a temperture above the melting point of the thermoplastic material.

The fabric material is preferably fed from one reel containing a roll of the fabric material and the prepared splint material is fed onto a take-up reel. Prior to being fed onto the take-up reel the prepared splint material is cooled, preferably by a waterspray.

Embodiments of the invention will now be described by way of example with reference to the accompanying drawings in which: -

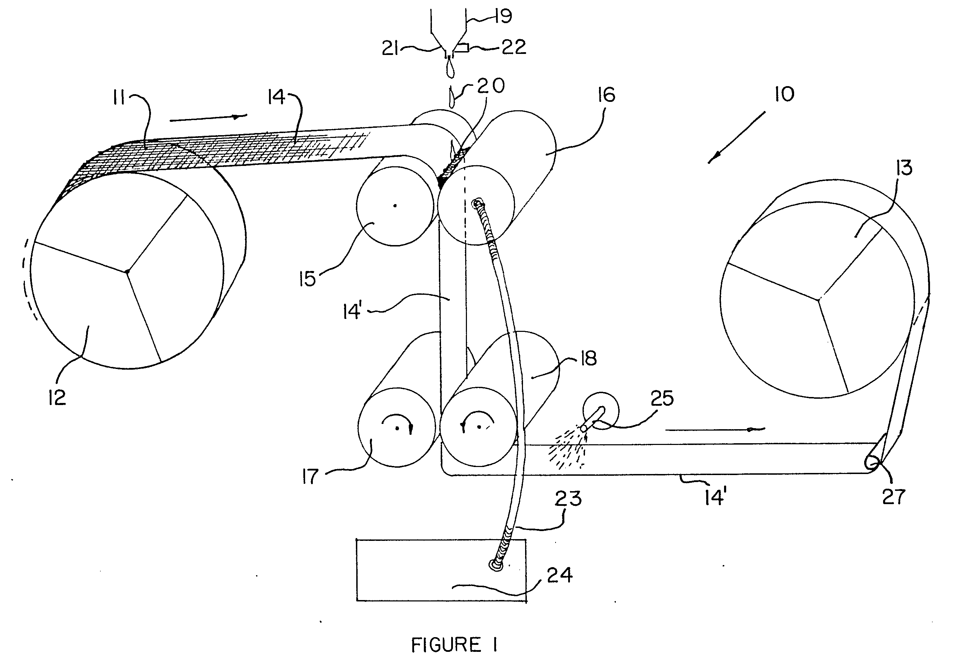

Figure 1 is a diagrammatic representation of an apparatus for preparing a thermoplastic orthopaedic splint material according to the invention; and

Figure 2 is a perspective view of part of a sheet of orthopaedic material according to the invention.

Referring now to the drawings wherein similar numerals have been used to indicate like parts, there is illustrated therein an apparatus generally indicated at 10 for the production of a thermoplastic orthopaedic splint material according to the invention. A roll of inert substrate fabric material 11 is held on a dispensing reel 12 and is fed to a take-up reel 13. The reels 12, 13 may be driven by respective motors (not shown) so that the fabric material or substrate 14 in the region between the reels 12, 13 may be tensioned. Clearly the reel 12 will rotate in a clockwise direction and reel 13 rotates in an anti-clockwise direction. The fabric material 14 passes between two stationary cyclindrical brass guide members 15, 16 and a pair of draw rollers 17, 18 as it travels from reel 12 to reel 13.

A mass of the thermoplastic material or resin in the form of polycaprolactone pellets (sold by Interox Chemicals Limited under the name CAPA) are extruded into a jacketed holding tank 19 heated to a temperature of approximately 65°C i.e. a temperature above the melting point of the polycaprolactone (PCL). Melted wax is added to the PCL in the tank 19 to comprise approximately 1% to 10% and more preferably approximately 5% by weight of the resin mixture in the tank 19 and the wax is mixed in conventional manner with the PCL. The resin mixture 20 is then discharged through an elongate nozzel 21 controlled by a valve 22, at a controlled rate, dependent upon the velocity of the fabric material 14. The resin mixture 20 is deposited onto the fabric material 14 in the region between the cylindrical guide members 15, 16 as shown. The cylindrical member 16 is hollow and is heated to a temperature above the melting point of the resin 20, by passing hot water through the member 16 via a conduit 23 from a water supply/heating a pump system 24. An outlet pipe (not shown) connected to the member 16 conveys the water from the member 16 either to a waste pipe (not shown) or returns it to the system 24.

The guide members 15, 16 are spaced apart slightly to a distance which determines the thickness of the coating of resin 20 on the fabric material 14. The wax in the resin mixture 20 provides the mixture with a consistency which enables the resin to be readily spread onto the fabric material 14. It has been found that a resin mixture 20 containing approximately 95% polycaprolactone and approximately 5% of a compatible wax provides an optimum consistency of the resin. Thus, as the fabric material 14 emerges from between the guide members 15, 16 it is coated with the resin 20 to a predetermined thickness and the fabric material 14 is effectively embedded in the resin 20.

The draw rollers 17, 18 serve to draw the coated substrate 14' downwards away from the members 15, 16 and the rollers 17, 18 which rotate towards each other, are also slightly spaced apart to further regulate the thickness of the coated substrate 14'. After passing through the draw rollers 17, 18 the coated substrated 14' is cooled to a temperature below the melting temperature of the resin 20 by spraying it with water from a cold water supply 25, after which it is stored on the take up reel 13, as the finished product. Once the coated substrate is sprayed with the cold water, and the resin has cooled to a temperature below its melting temperature, the resin becomes solid, but the coated substrate is sufficiently flexible to enable it to be wound onto the take-up wheel 13 via a guide member 27.

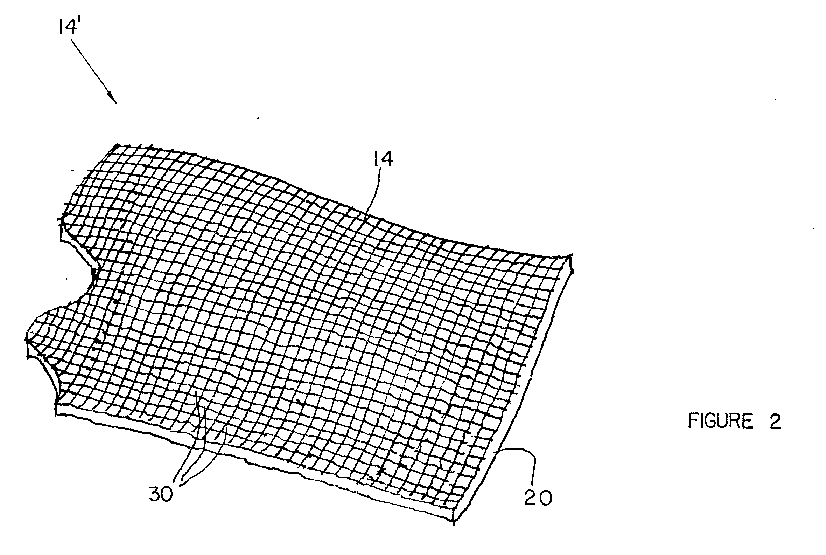

A portion of the coated substrate 14' as wound onto the reel 13 is illustrated in Figure 2 and comprises a layer of the substrate fabric material 14 embedded in the thermoplastic resin 20. The fabric substrate 14 is in the form of a textile netting, similar to net curtains, having a multitude of interstices 30 into which the resin becomes embedded as the fabric passes through the members 15, 16 and the rollers 17, 18.

When required for use the coated substrate 14' is simply cut to a desired shape and size and then immersed in hot water so that it becomes mouldable. The coated substrate is then moulded to the desired shape as a splint, for example for a patient with an injured or broken limb. The coated substrate is then cooled by quenching in cold water so that it becomes rigid in the moulded shape.

The primary advantages of the invention is that a thermoplastic orthopaedic splint material is provided at a minimal cost, and which is readily mouldable simply by immersing in hot water. The fabric substrate material provides the thermoplastic resin with sufficient viscosity without the need for expensive cross-linking of the resin.

## Claims

1. A thermoplastic orthopaedic splint material comprising a flexible inert substrate (14) embedded in a thermoplastic material (20).

2. A splint material as claimed in Claim 1, wherein the thermoplastic material (20) comprises a non-cross-linked polymer.

3. A splint material as claimed in Claim 1 or 2, wherein the substrate (14) comprises a fabric material.

4. A splint material as claimed in any preceding claim, wherein the thermoplastic material (20) comprises polycaprolactone.

5. A splint material as claimed in Claim 4, wherein the thermoplastic material (20) comprises approximately 90% to 99% by weight of polycaprolactone and approximately 1% to 10% by weight of a compatible wax.

6. A splint material as claimed in Claim 5, wherein the thermoplastic material comprises approximately 94% to 97% by weight of polycaprolactone and approximately 3% to 6% by weight of a compatible wax.

7. A process for the production of a thermoplastic orthopaedic splint material as claimed in any one of Claims 1 to 6, comprising the steps of, coating an inert flexible substrate (14) with a layer of thermoplastic material (20) which is at or above its melting temperature, and cooling the combined substrate and thermoplastic material (14') below the melting temperature of the thermoplastic material.

8. A process as claimed in Claim 7, wherein the thermoplasti material (20) is applied to a moving sheet (14) of the inert substrate.

9. An apparatus for the production of a thermoplastic orthopaedic splint material as claimed in any one of Claims 1 to 6, comprising means (15,16) for coating an inert flexible substrate (14) with a layer of thermoplastic material (20) which is at or above its melting temperature, and means for cooling (25) the combined substrate and thermoplastic material (14') below the melting temperature of the thermoplastic material.

10. An apparatus as claimed in Claim 9, wherein there is provided means (17,18) to move the substrate below a supply (19) of thermoplastic material (20), said substrate moving between a pair of guide members, the thermoplastic material being discharged onto the substrate in the region between the guide members.

FIGURE I

EP 0 338 815 A1

FIGURE 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 273 115 (HOLLAND et al.) * Whole document * | 1-4,7-10 | A 61 F 13/04 |
| Y | | 5,6 | |
| X | US-A-4 326 509 (USUKURA) * Abstract; figure; column 3, lines 50-56 * | 1-4,7-10 | |
| Y | US-A-4 454 873 (LAUFENBERG et al.) * Abstract; column 3, lines 2-5 * | 5,6 | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 12-07-1989 | SANCHEZ Y SANCHEZ J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)